(19) Europäisches Patentamt
European Patent Office
Office européen des brevets

(11) Veröffentlichungsnummer: **0 036 502**
**B1**

(12) # EUROPÄISCHE PATENTSCHRIFT

(45) Veröffentlichungstag der Patentschrift: **19.10.83**

(51) Int. Cl.³: **C 07 C 50/32**, C 07 C 46/00, A 61 K 31/12

(21) Anmeldenummer: **81101380.4**

(22) Anmeldetag: **25.02.81**

(54) **2-Alkyl-5-hydroxy-1,4-naphthochinone, Verfahren zu ihrer Herstellung sowie diese Verbindungen enthaltende Arzneimittel.**

(30) Priorität: **27.02.80 DE 3007367**

(43) Veröffentlichungstag der Anmeldung: **30.09.81 Patentblatt 81/39**

(45) Bekanntmachung des Hinweises auf die Patenterteilung: **19.10.83 Patentblatt 83/42**

(84) Benannte Vertragsstaaten: **AT BE CH DE FR GB IT LI LU NL SE**

(56) Entgegenhaltungen:
**US-A-2 572 946**
**US-A-3 578 686**

(73) Patentinhaber: **ZYMA GMBH, Zielstattstrasse 40, *D-8000 München 70 (DE)***

(72) Erfinder: **Wurm, Gotthard, Prof. Dr. Freie Universität Berlin, Institut für Pharmazie Königin-Luise-Strasse 2+4, 1-1000 Berlin 33 (DE)**

(74) Vertreter: **Patentanwälte Dipl.-Ing. A. Grünecker, Dr.-Ing. H. Kinkeldey, Dr.-Ing. W. Stockmair,, Dr. rer. nat. K. Schumann, Dipl.-Ing. P.H. Jakob, Dr. rer. nat. G. Bezold Maximilianstrasse 58, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 036 502 B1

2-Alkyl-5-hydroxy-1,4-naphthochinone, Verfahren zu ihrer Herstellung sowie diese Verbindungen enthaltende Arzneimittel

Die Erfindung betrifft 2-Alkyl-5-hydroxy-1,4-naphthochinone der allgemeinen Formel I

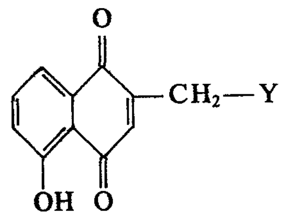

(I)

worin Y die Methyl-, Äthyl-, n-Propyl-, Isopropyl- oder tert.-Butylgruppe bedeutet, nämlich

2-Äthyl-5-hydroxy-1,4-naphthochinon,
2-Propyl-5-hydroxy-1,4-naphthochinon,
2-Propyl-5-hydroxy-1,4-naphthochinon,
2-n-Butyl-5-hydroxy-1,4-naphthochinon,
2-Isobutyl-5-hydroxy-1,4-naphthochinon und
2-Neopentyl-5-hydroxy-1,4-naphthochinon.

Die genannten Naphthochinone der allgemeinen Formel I sind neue Verbindungen mit wertvollen pharmakologischen Eigenschaften.

Diese Verbindungen können erfindungsgemäß dadurch hergestellt werden, daß man

(1) 1-Hydroxy-2-acyl-5-methoxynaphthalin der allgemeinen Formel II

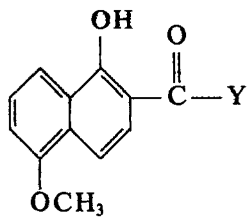

(II)

worin Y die in Anspruch 1 angegebene Bedeutung hat, mit Halogenameisensäureester im Molverhältnis von mindestens 1 : 1 in Gegenwart einer Base umsetzt und anschließend mit einer Borwasserstoffverbindung reduziert,

(2) das so erhaltene 1-Hydroxy-2-alkyl-5-methoxynaphthalin der allgemeinen Formel III

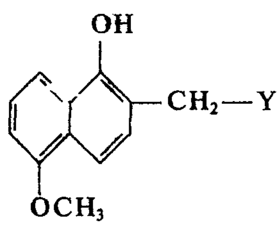

(III)

worin Y die vorstehend angegebene Bedeutung hat, entweder nach Verfahrensvariante A

α) mit Kaliumnitrosodisulfonat oxidiert oder
β) mit Sauerstoff photooxidiert und

das so erhaltene 2-Alkyl-5-methoxy-1,4-naphthochinon der allgemeinen Formel IV

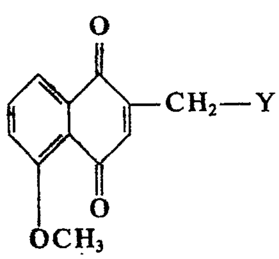

(IV)

worin Y die vorstehend angegebene Bedeutung hat, mit einem Ätherspaltungsreagenz entmethyliert

oder nach Verfahrensvariante B

mit einem Ätherspaltungsreagenz entmethyliert und das so erhaltene 1,5-Dihydroxy-2-alkylnaphthalin der allgemeinen Formel V

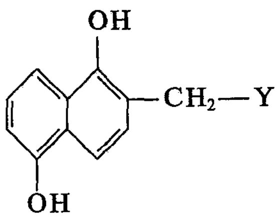

(V)

worin Y die vorstehend angegebene Bedeutung hat,

α) mit Kaliumnitrosodisulfonat oxidiert oder
β) mit Sauerstoff photooxidiert.

Die Umsetzung von 1-Hydroxy-2-acyl-5-methoxynaphthalin der allgemeinen Formel II zur Herstellung von 1-Hydroxy-2-alkyl-5-methoxynaphthalin der allgemeinen Formel III kann analog dem Verfahren von Minami und Kijima, Chem. Pharm. Bull., Bd. 27, S. 1490–1494 (1979), erfolgen. Dabei ist es bevorzugt, als Halogenameisensäureester den Chlorameisensäureäthylester einzusetzen, um so ein Äthoxycarbonylderivat herzustellen. Zweckmäßigerweise wird die bei dieser Reaktion verwendete Base, bezogen auf die Verbindung der allgemeinen Formel II, in einem Molverhältnis von mindestens 1 : 1 verwendet. Als Base wird vorzugsweise Triäthylamin eingesetzt. Die Umsetzung erfolgt zweckmäßigerweise in einem organischen Lösungsmittel, wobei Tetrahydrofuran bevorzugt ist. Die Reduktion zum 1-Hydroxy-2-alkyl-5-methoxynaphthalin der allgemeinen Formel III erfolgt schließlich mit einer Borwasserstoffverbindung, beispielsweise Natriumborhydrid, Lithiumborhydrid oder Demethylaminboran, wobei Natriumborhydrid bevorzugt eingesetzt wird.

Die so erhaltene Verbindung der allgemeinen Formel III kann erfindungsgemäß auf verschiedene Weise weiter umgesetzt werden. Entweder kann zuerst zum 1,4-Naphthochinonderivat oxidiert und anschließend die Äthergruppe in der 5-Stellung gespalten werden, oder es kann zuerst die Äthergruppe gespalten und dann zum 1,4-Naphthochinonderivat oxidiert werden. Diese verschiedenen Umsetzungsmöglichkeiten sind unter der erfindungsgemäßen Verfahrensstufe (2) zusammengefaßt.

Bei der Oxidation mit Kaliumnitrosodisulfonat ist es bevorzugt, daß das Kaliumnitrosodisulfonat mit dem zu oxidierenden 1-Hydroxy-2-alkyl-5-methoxynaphthalin der allgemeinen Formel III bzw. dem 1,5-Dihydroxy-2-alkylnaphthalin der allgemeinen Formel V in einem Molverhältnis von mindestens 2 : 1 umgesetzt wird. Diese Umsetzung kann in wäßriger Lösung erfolgen, wobei die Lösung zusätzlich einen Puffer, wie Kaliumdihydrogenphosphat, enthalten kann.

Die Photooxidation mit Sauerstoff kann analog der Methode von J. Griffiths, J. C. S. Chem. Comm. S. 676–677 (1976) erfolgen. Die zu oxidierende Substanz wird hierbei in einem organischen Lösungsmittelgemisch gelöst und mit sichtbarem Licht bestimmter Wellenlänge, beispielsweise unter Verwendung von Wolframlampen, in Gegenwart eines Farbstoff-Sensibilisators bestrahlt. Erfindungsgemäß ist es bevorzugt, die Bestrahlung in Gegenwart von Methylenblau als Sensibilisator vorzunehmen.

Als Ätherspaltungsreagenz werden bevorzugt Jodwasserstoffsäure, Pyridinhydrochlorid, Bortribromid oder Aluminiumtrichlorid eingesetzt. Weiterhin ist es bevorzugt, bei der Spaltung von 1-Hydroxy-2-alkyl-5-methoxynaphthalin der allgemeinen Formel III als Ätherspaltungsreagenz Bortribromid und bei der Spaltung von 2-Alkyl-5-methoxy-1,4-naphthochinon der allgemeinen Formel IV Aluminiumtrichlorid als Ätherspaltungsreagenz zu verwenden, da unter Verwendung dieser Reagenzien die jeweilige Ätherspaltung besonders glatt verläuft.

Bei der Oxidation von 1,5-Dihydroxy-2-alkylnaphthalin der allgemeinen Formel V mit Kaliumnitrosodisulfonat entstehen neben dem erwünschten 2-Alkyl-5-hydroxy-1,4-naphthochinon der allgemeinen Formel I bis maximal 10% des isomeren 5-Hydroxy-6-alkyl-1,4-naphthochinons, und bei der Photooxidation von 1,5-Dihydroxy-2-alkylnaphthalin der allgemeinen Formel V entstehen bis etwa 20% des vorgenannten Isomeren. Das Isomere kann auf einfache Weise vom erwünschten Endprodukt chromatographisch abgetrennt werden.

Das im erfindungsgemäßen Verfahren als Ausgangsverbindung eingesetzte 1-Hydroxy-2-acyl-5-methoxynaphthalin der allgemeinen Formel II wird zweckmäßigerweise dadurch hergestellt, daß 1-Alkylcarboxy-5-methoxynaphthalin der allgemeinen Formel VI

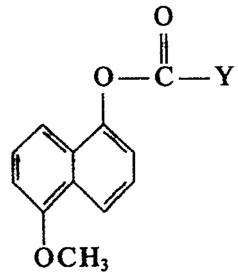

(VI)

worin Y die vorstehend genannte Bedeutung hat,
einer Photo-Fries-Umlagerung unterworfen wird. Die Reaktion kann durch Bestrahlen der Verbindung der allgemeinen Formel VI in alkoholischer, beispielsweise methanolischer, Lösung mit ultraviolettem Licht erfolgen. Weiterhin kann die Umlagerung der Verbindung der allgemeinen Formel VI auch unter Verwendung von Bortrifluorid durchgeführt werden.

Das für die vorgenannte Umlagerung als Ausgangsverbindung eingesetzte 1-Alkylcarboxy-5-methoxynaphthalin kann durch Acylierung von 1-Hydroxy-5-methoxynaphthalin der Formel VII

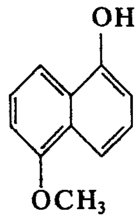

(VII)

mit einem Acylierungsmittel, wie Acetanhydrid, in gegenwart einer Base, wie Pyridin, hergestellt werden.

Die Herstellung der Verbindung der Formel VII kann schließlich durch partielle Methylierung von 1,5-Dihydroxynaphthalin der Formel VIII

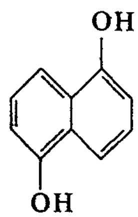

(VIII)

oder, analog der Methode von H. Rapoport, J. Org. Chem., Bd. 44, S. 2153 (1979), durch partielle Ätherspaltung von 1,5-Dimethoxynaphthalin der Formel IX

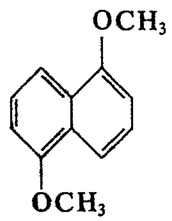

(IX)

mit einer Aufschlämmung von Natriumhydrid und Äthanthiol erfolgen. Diese Reaktion wird zweckmäßigerweise in einem organischen Lösungsmittel, beispielsweise Dimethylformamid durchgeführt.

Die erfindungsgemäßen Verbindungen der allgemeinen Formel I haben überraschenderweise ausgezeichnete Prostaglandin-synthetase-hemmende sowie antiallergische und insbesondere antianaphylaktische Eigenschaften. Sie dienen daher zur Behandlung und Bekämpfung von Entzündungen sowie von allergischen, insbesondere anaphylaktischen Reaktionen.

Die Erfindung betrifft somit weiterhin die Verwendung der erfindungsgemäßen Verbindungen der allgemeinen Formel I zur Behandlung und Bekämpfung von Entzündungen sowie von allergischen, insbesondere anaphylaktischen Reaktionen. So beträgt beispieslweise die durch 2-Äthyl-5-hydroxy-1,4-naphthochinon hervorgerufene Prostaglandinsynthetase-Hemmung in vitro bei einer Konzentration von $10^{-3}$ Mol/l 87%. Die Bestimmung der Prostaglandinsynthetase-Hemmung wurde nach J. Baumann, F. v. Bruchhausen u. G. Wurm, Naunym-Schmiedeberg's Arch. Pharmacol. 307, 73 (1979) und j. Baumann, G. Wurm u. F. v. Bruchhausen, Arch. Pharm. (Weinheim) 313, 330 (1980) durchgeführt.

Weiterhin betrifft die Erfindung Arzneimittel, die gekennzeichnet sind durch einen Gehalt an mindestens einer erfindungsgemäßen Verbindung der allgemeinen Formel I als Wirkstoff und üblichen, pharmakologisch verträglichen Trägerstoffen und/oder Verdünnungsmitteln. Die erfindungsgemäßen Arzneimittel können beispielsweise oral in Form von Tabletten, Dragees oder Lösungen sowie in Form von Injektionslösungen und Suppositorien vorliegen.

Die Beispiele erläutern die Erfindung.

In den Beispielen 1 bis 4 sind die verschiedenen erfindungsgemäßen Varianten zur Herstellung von 2-Äthyl-5-hydroxy-1,4-naphthochinon beschrieben.

In den Beispielen 5 bzw. 6 ist die Herstellung der Ausgangsverbindungen 1-Hydroxy-2-acetyl-5-methoxynaphthalin bzw. 1-Acetoxy-5-methoxynaphthalin beschrieben.

Die Herstellung der übrigen erfindungsgemäßen Verbindungen erfolgt in analoger Weise.

## Beispiel 1

1. 10,8 g (0,05 Mol) 1-Hydroxy-2-acetyl-5-methoxynaphthalin werden zunächst mit 6,0 g (0,06 Mol) Triäthylamin und 6,5 g (0,06 Mol) Chlorameisensäureäthylester in 70 ml Tetrahydrofuran bei 0°C umgesetzt. Nach einer Reaktionszeit von 1 Stunde wird filtriert und das Filtrat bei 10°C mit 7,9 g (0,2 Mol) Natriumborhydrid in 100 ml Wasser versetzt. Nach 2 Stunden wird der Ansatz mit Salzsäure angesäuert und der Niederschlag mit Wasser gewaschen und anschließend getrocknet. Man erhält 1-Hydroxy-2-äthyl-5-methoxynaphthalin in Form farbloser Nadeln.
   Ausbeute 85 – 90%,
   Fp 85 – 86°C (aus Äthanol).
2. 1,01 g (0,005 Mol) des nach 1. hergestellten 1-Hydroxy-2-äthyl-5-methoxynaphthalins in 50 ml Methanol werden mit 3 g (0,011 Mol) Kaliumnitrosodisulfonat in 250 ml Wasser und 50 ml $\frac{m}{6}$ Kaliumdihydrogenphosphatlösung versetzt. Nach 2 Stunden wird das Reaktionsprodukt mit Chloroform extrahiert, die organische Phase mit Natriumsulfat getrocknet und der Rückstand bei 70°C und 13,3 Pa sublimiert. Man erhält 2-Äthyl-5-methoxy-1,4-naphthochinon der allgemeinen Formel IV in Form gelber Nadeln.
   Ausbeute 60 – 70%,
   Fp 83 – 85°C.
3. 1 g des nach 2. hergestellten 2-Äthyl-5-methoxy-1,4-naphthochinons und 3 g Aluminiumtrichlorid werden 2 Stunden bei 20°C in 100 ml Methylenchlorid gerührt und anschließend mit 200 ml Eiswasser versetzt. Es wird mit Chloroform extrahiert, die organische Phase mit Natriumsulfat getrocknet und der Rückstand bei 40°C und 13,3 Pa sublimiert. Man erhält das erwünschte 2-Äthyl-5-hydroxy-1,4-naphthochinon in Form gelber Nadeln.
   Ausbeute 65 – 70%,
   Fp 97°C.

## Beispiel 2

1. Analog der Verfahrensstufe 1 des Beispiels 1 wird 1-Hydroxy-2-äthyl-5-methoxynaphthalin hergestellt.
2. 3 g 1-Hydroxy-2-äthyl-5-methoxynaphthalin werden in 20 ml Methylenchlorid bei – 10°C mit 25 g Bortribromid versetzt und dann auf Kühlschranktemperatur gehalten. Nach 12 Stunden werden Reagenz und Lösungsmittel bei 20°C abdestilliert, der Rückstand mit Eiswasser versetzt und das Reaktionsprodukt mit Chloroform extrahiert. Man erhält 1,5-Dihydroxy-2-äthylnaphthalin in Form farbloser Nadeln.
   Ausbeute 80 – 85%,
   Fp 166 – 167°C.
3. 0,94 g (0,005 Mol) 1,5-Dihydroxy-2-äthylnaphthalin werden analog der Verfahrensstufe 2 des Beispiels 1 unter Verwendung von Kaliumnitrosodisulfonat oxidiert. Die Chloroformphase enthält etwa 10% des Isomeren 5-Hydroxy-6-äthyl-1,4-naphthochinon (rot-braune Nadeln, Fp 65 – 67°C), das auf chromatographischem Weg mit Benzol an Kieselgel vom erwünschten 2-Äthyl-5-hydroxy-1,4-naphthochinon abgetrennt wird. Das so gewonnene Endprodukt ist mit dem nach Beispiel 1 gewonnenen Produkt identisch.

## Beispiel 3

1. Analog der Verfahrensstufe 1 des Beispiels 1 wird 1-Hydroxy-2-äthyl-5-methoxynaphthalin hergestellt.
2. 3 g 1-Hydroxy-2-äthyl-5-methoxynaphthalin und 0,3 g Methylenblau werden in 1000 ml Methanol

gelöst. Diese Lösung wird 12 Stunden mit 4 Wolframlampen (je Lampe 300 Watt) bei 15° C unter Durchleiten von Sauerstoff bestrahlt. Nach Abziehen des Methanols wird das gebildete 2-Äthyl-5-methoxy-1,4-naphthochinon in 200 ml Methylenchlorid aufgenommen.

3. Die nach 2. gewonnene Lösung wird mit 7,5 g Aluminiumtrichlorid versetzt und 2 Stunden bei 20° C gerührt. Anschließend wird mit 400 ml Eiswasser versetzt und mit Chloroform extrahiert. Die organische Phase wird mit Natriumsulfat getrocknet, der Rückstand in Benzol aufgenommen und an Kieselgel mit Benzol chromatographiert. Das gelb gefärbte Eluat wird eingeengt und bei 40° C und 13,3 Pa sublimiert. Man erhält 2-Äthyl-5-hydroxy-1,4-naphthochinon in Form gelber Nadeln.
Ausbeute 50%.
Die Verbindung ist mit der nach Beispiel 1 hergestellten Verbindung identisch.

Beispiel 4

1. Analog der Verfahrensstufe 2 des Beispiels 2 wird 1,5-Dihydroxy-2-äthylnaphthalin hergestellt.
2. 0,94 g (0,005 Mol) 1,5-Dihydroxy-2-äthylnaphthalin werden analog der Verfahrensstufe 2 des Beispiels 3 photooxidiert. Der Ansatz enthält neben dem erwünschten 2-Äthyl-5-hydroxy-1,4-naphthochinon etwa 20% des isomeren 5-Hydroxy-6-äthyl-1,4 naphthochinons (rot-braune Nadeln, Fp 65 – 67° C), das an Kieselgel mit Benzol abgetrennt werden kann. Das so gereinigte 2-Äthyl-5-hydroxy-1,4-naphthochinon weist mit dem nach Beispiel 1 hergestellten Produkt identische Eigenschaften auf.

Beispiel 5

10 g 1-Acetoxy-5-methoxynaphthalin werden in 1000 ml Methanol gelöst und 12 Stunden bei 15° C mit einem Quecksilberhochdurckbrenner (150 Watt) unter Stickstoff bestrahlt. Nach Entfernung des Methanols wird der Rückstand in Chloroform gelöst und an Kieselgel chromatographiert. Nach Entfernung des Lösungsmittels wird 1-Hydroxy-2-acetyl-5-methoxynaphthalin in Form gelber Nadeln erhalten.
Ausbeute 70 – 75%.
Fp 121 – 122° C (aus n-Butanol).

Beispiel 6

10 g 1-Hydroxy-5-methoxynaphthalin werden mit 10 ml Pyridin und 90 ml Acetanhydrid auf 100° C erwärmt und anschließend 12 Stunden bei Raumtemperatur belassen. Daran anschließend wird der Ansatz auf 1000 g gekörntes Eis gegossen. Das ausgefallene 1-Acetoxy-5-methoxynaphthalin wird nach 12 Stunden abgesaugt, mit Wasser gewaschen und an der Luft getrocknet. Zur Reinigung des Reaktionsproduktes wird dieses in Chloroform gelöst und über Kiegelgel filtriert. Nach Entfernung des Lösungsmittels werden farblose Nadeln erhalten.
Ausbeute 90 – 95%,
Fp 66 – 68° C (aus Äthanol).

**Patentansprüche**

1. 2-Alkyl-5-hydroxy-1,4-naphthochinone der allgemeinen Formel I

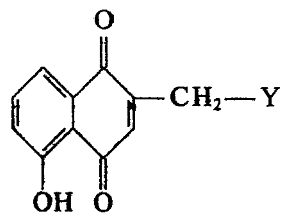

(I)

worin Y die Methyl-, Äthyl-, n-Propyl-, Isopropyl- oder tert.-Butylgruppe bedeutet.

2. Verfahren zur Herstellung der Verbindungen nach Anspruch 1, dadurch gekennzeichnet, daß man

(1) 1-Hydroxy-2-acyl-5-methoxynaphthalin der allgemeinen Formel II

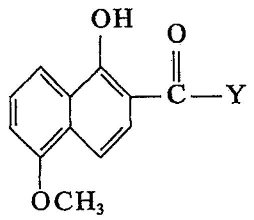

(II)

worin Y die in Anspruch 1 angegebene Bedeutung hat, mit Halogenameisensäureester im Molverhältnis von mindestens 1 : 1 in Gegenwart einer Base umsetzt und anschließend mit einer Borwasserstoffverbindung reduziert,

(2) das so erhaltene 1-Hydroxy-2-alkyl-5-methoxynaphthalin der allgemeinen Formel III

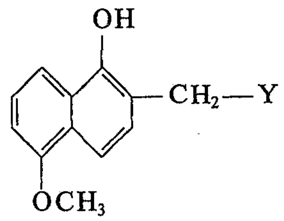

(III)

worin Y die vorstehend angegebene Bedeutung hat, entweder nach Verfahrensvariante A

α) mit Kaliumnitrosodisulfonat oxidiert oder
β) mit Sauerstoff photooxidiert und

das so erhaltene 2-Alkyl-5-methoxy-1,4-naphthochinon der allgemeinen Formel IV

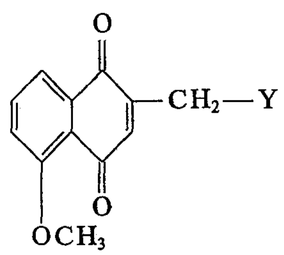

(IV)

worin Y die vorstehend angegebene Bedeutung hat, mit einem Ätherspaltungsreagenz entmethyliert

oder nach Verfahrensvariante B

mit einem Ätherspaltungsreagenz entmethyliert und das so erhaltene 1,5-Dihydroxy-2-alkylnaphthalin der allgemeinen Formel V

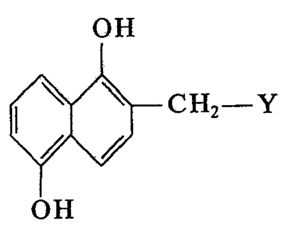

(V)

worin Y die vorstehend angegebene Bedeutung hat,

α) mit Kaliumnitrosodisulfonat oxidiert oder
β) mit Sauerstoff photooxidiert.

3. Verfahren nach Anspruch 2, dadurch gekennzeichnet, daß man in der Verfahrensstufe (1) als Halogenameisensäureester Chlorameisensäureäthylester einsetzt.

4. Verfahren nach einem der Ansprüche 2 und 3, dadurch gekennzeichnet, daß man pro Mol der Verbindung der Formel II mindestens 1 Mol Base einsetzt.

5. Verfahren nach einem der Ansprüche 2 bis 4, dadurch gekennzeichnet, daß man in der

Verfahrensstufe (1) als Base Triäthylamin einsetzt.

6. Verfahren nach einem der Ansprüche 2 bis 5, dadurch gekennzeichnet, daß man in der Verfahrensstufe (1) die Umsetzung mit Halogenameisensäureester in Tetrahydrofuran als Lösungsmittel durchführt.

7. Verfahren nach einem der Ansprüche 2 bis 6, dadurch gekennzeichnet, daß man in der Verfahrensstufe (1) als Borwasserstoffverbindung Natriumborhydrid einsetzt.

8. Verfahren nach Anspruch 2, dadurch gekennzeichnet, daß man in der Verfahrensstufe (2) das Kaliumnitrosodisulfonat mit der zu oxidierenden Verbindung der allgemeinen Formel III bzw. der Verbindung der allgemeinen Formel V in einem Molverhältnis von mindestens 2 : 1 umsetzt.

9. Verfahren nach Anspruch 2, dadurch gekennzeichnet, daß man die Photooxidation in der Verfahrensstufe (2) in Gegenwart von Methylenblau durchführt.

10. Verfahren nach Anspruch 2, dadurch gekennzeichnet, daß man in der Verfahrensstufe (2) als Ätherspaltungsreagenz Jodwasserstoffsäure, Pyridinhydrochlorid, Bortribromid oder Aluminiumtrichlorid einsetzt.

11. Verfahren nach Anspruch 10, dadurch gekennzeichnet, daß man als Ätherspaltungsreagenz zur Spaltung von 1-Hydroxy-2-alkyl-5-methoxynaphthalin der allgemeinen Formel III Bortribromid bzw. zur Spaltung von 1-Alkyl-5-methoxy-1,4-naphthochinon der allgemeinen Formel IV Aluminiumtrichlorid einsetzt.

12. Verfahren nach Anspruch 2, dadurch gekennzeichnet, daß man das als Ausgangsverbindung eingesetzte 1-Hydroxy-2-acyl-5-methoxynaphthalin der allgemeinen Formel II aus 1-Alkylcarboxy-5-methoxynaphthalin der allgemeinen Formel VI

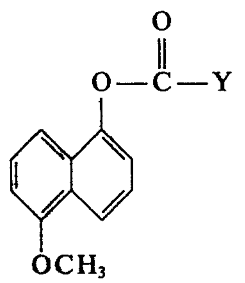

(VI)

worin Y die vorstehend angegebene Bedeutung hat, nach der Photo-Fries-Reaktion herstellt.

13. Verfahren nach Anspruch 12, dadurch gekennzeichnet, daß man das 1-Alkylcarboxy-5-methoxynaphthalin der allgemeinen Formel VI durch Acylierung von 1-Hydroxy-5-methoxynaphthalin der Formel VII

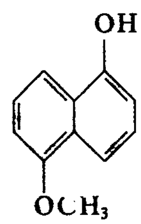

(VII)

herstellt.

14. Verfahren nach Anspruch 13, dadurch gekennzeichnet, daß man das 1-Hydroxy-5-methoxynaphthalin der Formel VII durch partielle Methylierung von 1,5-Dihydroxynaphthalin der Formel VIII

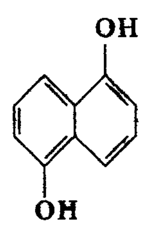

(VIII)

oder durch partielle Ätherspaltung von 1,5-Dimethoxynaphthalin der Formel IX

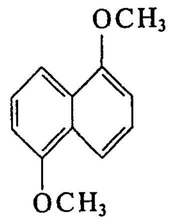

(IX)

mit Natriumhydrid und Äthanthiol herstellt.

15. Arzneimittel, gekennzeichnet durch einen Gehalt an mindestens einer Verbindung nach Anspruch 1 und üblichen, pharmakologisch verträglichen Trägerstoffen und/oder Verdünnungsmitteln.

16. Verbindungen nach Anspruch 1 zur Verwendung in einem Verfahren zur Behandlung von allergischen, insbesondere anaphylaktischen Reaktionen, sowie von Entzündungen.

**Claims**

1. 2-Alkyl-5-hydroxy-1,4-naphthoquinones of the general formula I

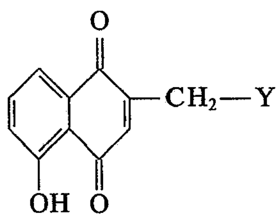

(I)

wherein Y denotes the methyl, ethyl, n-propyl, isopropyl or tert.-butyl group.

2. Process for the preparation of the compounds according to Claim 1, characterised in that (1) 1-hydroxy-2-acyl-5-methoxynaphthalene of the general formula II

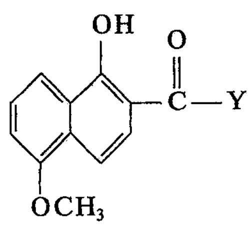

(II)

wherein Y has the meaning indicated in Claim 1, is reacted with a halogenoformic ester in a molar ratio of at least 1 : 1 in the presence of a base, and is then reduced with a borohydride compound, (2) the 1-hydroxy-2-alkyl-5-methoxynaphthalene of the general formula III thus obtained

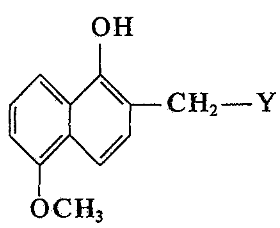

(III)

wherein Y has the meaning indicated above, is either, by process variant A,

*α*) oxidised with potassium nitrosodisulphonate or

*β*) photooxidised with oxygen and

the 2-alkyl-5-methoxy-1,4-naphthoquinone of the general formula IV thus obtained

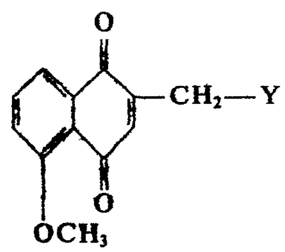

(IV)

wherein Y has the meaning indicated above, is demethylated with a reagent for ether cleavage or is, by process variant B, demethylated with a reagent for ether cleavage and the 1,5-dihydroxy-2-alkylnaphthalene of the general formula V thus abtained

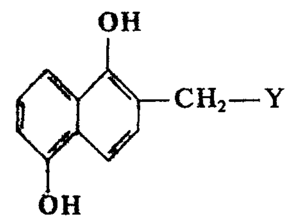

(V)

wherein Y has the meaning indicated above, is

α) oxidised with potassium nitrosodisulphonate or
β) photooxidised with oxygen.

3. Process according to Claim 2, characterised in that ethyl chloroformate ist employed as the halogenoformic ester in process step (1).

4. Process according to one of Claims 2 and 3, characterised in that at least 1 mole of base is employed for each mole of the compound of the formula II.

5. Process according to one of Claims 2 to 4, characterised in that the base employed in process step (1) is triethylamine.

6. Process according to one of Claims 2 to 5, characterised in that the reaction with a halogenoformic ester in process step (1) is carried out in tetrahydrofuran as the solvent.

7. Process according to one of Claims 2 to 6, characterised in that sodium borohydride is employed in process step (1) as the borohydride compound.

8. Process according to Claim 2, characterised in that the potassium nitrosodisulphonate in process step (2) is reacted with the compound to be oxidised, of the general formula III of the compound of the general formula V in a molar ratio of at least 2 : 1.

9. Process according to Claim 2, characterised in that the photooxidation in process step (2) is carried out in the presence of methylene blue.

10. Process according to Claim 2, characterised in that hydriodic acid, pyridine hydrochloride, boron tribromide or aluminium trichloride is employed in process step (2) as the reagent for ether cleavage.

11. Process according to Claim 10, characterised in that, as the reagent for ether cleavage, boron trobromide is employed to cleave 1-hydroxy-2-alkyl-5-methoxynaphthalene of the general formula III and aluminium trichloride is employed to cleave 1-alkyl-5-methoxy-1,4-naphthoquinone of the general formula IV.

12. Process according to Claim 2, characterised in that the 1-hydroxy-2-acyl-5-methoxynaphthalene of the general formula II employed as the starting compound is prepared from 1-alkylcarboxy-5-methoxynaphthalene of the general formula VI

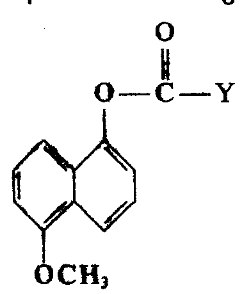

(VI)

wherein Y has the meaning indicated above, by the photo-Fries reaction.

13. Process according to Claim 12, characterised in that the 1-alkylcarboxy-5-methoxynaphthalene of the general formula VI is prepared by acylation of 1-hydroxy-5-methoxynaphthalene of the formula VII

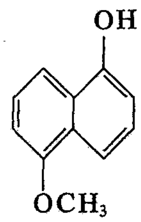

(VII)

14. Process according to Claim 13, characterised in that the 1-hydroxy-5-methoxynaphthalene of the formula VII is prepared by partial methylation of 1,5-dihydroxy-naphthalene of the formula VIII

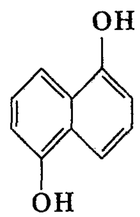

(VIII)

or by partial ether cleavage of 1,5-dimethoxynaphthalene of the formula IX

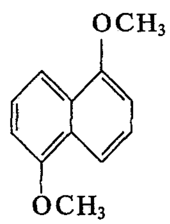

(IX)

using sodium hydride and ethanethiol.

15. Medicament characterised by a content of at least one compound according to Claim 1 and customary pharmacologically tolerated vehicles and/or diluents.

16. Compounds according to Claim 1 for use in a procedure for the treatment of allergic, especially anaphylatic reactions and of inflammations.

**Revendications**

1. 2-alkyl-5-hydroxy-1,4-naphthoquinones répondant à la formule générale I

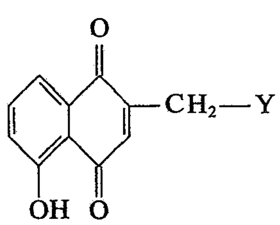

(I)

dans laquelle Y désigne les groupes méthyle, éthyle, n-propyle, isopropyle ou tert-butyle.

2. Procédé de fabrication des composés suivant la revendication 1, caractérisé en ce que

(1) on fait réagir un 1-hydroxy-2-acyl-5-méthoxynaphthalène répondant à la formule générale II

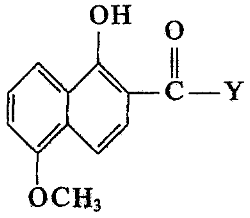

(II)

dans laquelle Y a la signification indiquée dans la revendication 1,
avec un ester halogénoformique, dans un rapport molaire d'au moins 1 : 1, en présence d'une base, et en ce qu'on le rédiut ensuite avec un composé hydrogéné du bore,

(2) on soumet le 1-hydroxy-2-alkyl-5-méthoxynaphthalène ainsi obtenu, répondant à la formule générale III

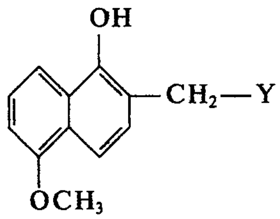

(III)

dans laquelle Y a la signification précédemment indiquée,
soit dans la variante A en procédant

α) à une oxydation par le nitrosodisulfonate de potassium ou
β) à une photo-oxydation par l'oxygène, et on déméthyle la 2-alkyl-5-méthoxy-1,4-naphthoquinone répondant à la formule générale IV

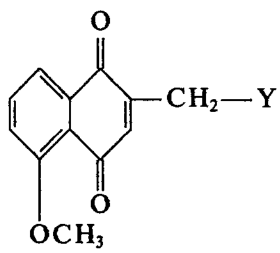

(IV)

dans laquelle Y a la signification précédemment indiquée, avec un réactif de coupure des éthers,

soit dans la variante B

du procédé à une déméthylation par un réactif de coupure des éthers, et on soumet le 1,5-dihydroxy-2-alkylnaphthalène obtenu répondant à la formule générale V

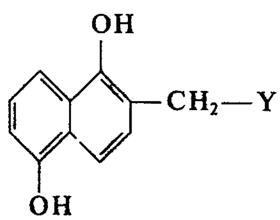

(V)

dans laquelle Y a la signification précédemment indiquée,

α) à une oxydation par le nitrosodisulfonate de potassium, ou
β) à une photo-oxydation par l'oxygène.

3. Procédé souvant la revendication 2, caractérisé en ce que dans le stade (1) du procédé, on utilise comme ester halogénoformique du chlorformiate d'éthyle.

4. Procédé suivant l'une quelconque des revendications 2 et 3, caractérisé en ce qu'on utilise, par mole du composé répondant à la formule II, au moins une mole de base.

5. Procédé suivant l'une quelconque des revendications 2 à 4, caractérisé en ce que dans le stade (1) du procédé, on utilise comme base la triéthylamine.

6. Procédé suivant l'une quelconque des revendications 2 à 5, caractérisé en ce que, dans le stade (1) du procédé, on effectue la réaction avec l'ester halogénoformique dans du tétrahydrofurane comme solvant.

7. Procédé suivant l'une quelconque des revendications 2 à 6, caractérisé en ce que dans le stade (1) du procédé, on utilise comme composé hydrogéné du bore du borohydrure de sodium.

8. Procédé suivant la revendication 2, caractérisé en ce que le stade (2) du procédé, on fait réagir le nitrosodisulfonate de potassium avec le composé à oxyder répondant à la formule générale III ou le composé répondant à la formule générale V dans un rapport molaire d'au moins 2 : 1.

9. Procédé suivant la revendication 2, caractérisé en ce que dans le stade (2) du procédé, on effectue la photo-oxydation en présence de bleu de méthylène.

10. Procédé suivant la revendication 2, caractérisé en ce que dans le stade (2) du procédé, on utilise comme téactif de coupure des éthers de l'acide iodhydrique, de l'hydrochlorure de pyridine, du tribromure de bore ou du trichlorure d'aluminium.

11. Procédé suivant la revendication 10, caractérisé en ce que on utilise comme réactif de coupure des éthers, pour la coupure du 1-hydroxy-2-alkyl-5-méthoxynaphthalène répondant à la formule générale III, du tribromure de bore, ou pour la coupure de la 1-alkyl-5-méthoxy-1,4-naphthoquinone répondant à la formule générale IV, du trichlorure d'aluminium.

12. Procédé suivant la revendication 2, caractérisé en ce qu'on prépare le 1-hydroxy-2-acyl-5-méthoxynaphthalène répondant à la formule générale II utilisé comme produit de départ, à partir du 1-alkylcarboxy-5-méthoxynaphthalène répondant à la formule générale VI

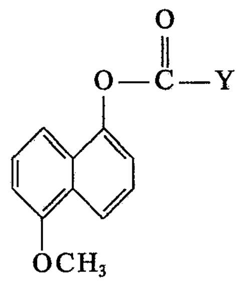

(VI)

dans laquelle Y a la signification précédemment indiquée, selon la photo-réaction de Fries.

13. Procédé suivant la revendication 12, caractérisé en ce qu'on prépare le 1-alkylcarboxy-5-méthoxynaphthalène répondant à la formule générale VI par acylation du 1-hydroxy-5-méthoxynaphthalène répondant à la formule VII

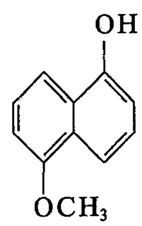

(VII)

14. Procédé suivant la revendication 13, caractérisé en ce qu'on prépare le 1-hydroxy-5-méthoxynaphthalène répondant à la formule VII par méthylation partielle du 1,5-dihydroxynaphthalène à la formule VIII

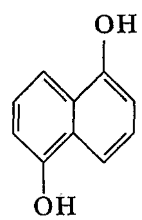

(VIII)

ou par coupure partielle de l'éther du 1,5-diméthoxynaphthalène répondant à la formule IX

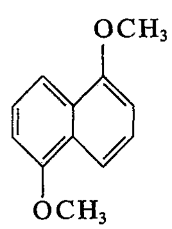

(IX)

avec de l'hydrure de sodium et de l'éthanethiol.

15. Médicament, caractérisé en ce qu'il contient au moins un composé suivant la revendication 1 et des supports et/ou diluants ordinaires, pharmacologiquement acceptables.

16. Composés suivant la revendication 1 pour l'utilisation dans un procédé pour le traitement de réactions allergiques, en particulier anaphylactiques, ainsi que d'inflammations.